(19) Europäisches Patentamt
European Patent Office
Office européen des brevets

(11) Numéro de publication: **0 080 411**
**B1**

(12) # FASCICULE DE BREVET EUROPÉEN

(45) Date de publication du fascicule du brevet: **22.07.87**

(51) Int. Cl.⁴: **G 01 N 33/84**, G 01 N 31/22, A 61 K 49/00

(21) Numéro de dépôt: **82402103.4**

(22) Date de dépôt: **18.11.82**

(54) **Indicateur coloré pour la détermination de l'hypoacidité de la muqueuse gastrique au cours de la gastroscopie.**

(30) Priorité: **20.11.81 FR 8121739**

(43) Date de publication de la demande: **01.06.83 Bulletin 83/22**

(45) Mention de la délivrance du brevet: **22.07.87 Bulletin 87/30**

(84) Etats contractants désignés: **AT BE CH DE FR GB IT LI LU NL SE**

(56) Documents cité:
**BE-A-685 684**
**FR-A-2 409 743**
**US-A-3 904 373**
**US-A-4 056 609**

(73) Titulaire: **SOCIETE D'ETUDES SCIENTIFIQUES ET INDUSTRIELLES DE L'ILE- DE- FRANCE, 46, Boulevard de Latour- Maubourg, F-75340 Paris Cedex 07 (FR)**

(72) Inventeur: **Besançon, François, 14 Boulevard Emile Augier, F-75016 Paris (FR)**
Inventeur: **Chast, François, 1, Place du Parvis Notre- Dame, F-75004 Paris (FR)**

(74) Mandataire: **Gallochat, Alain, SOCIETE D'ETUDES SCIENTIFIQUES ET INDUSTRIELLES DE L'ILE DE FRANCE 46, Boulevard de Latour Maubourg, F-75340 Paris Cedex 07 (FR)**

**EP 0 080 411 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La présente invention concerne un agent de diagnostic pour la détermination dé l'hypoacidité de la muqueuse gastrique en cours de la gastroscopie.

Une telle détermination de l'hypoacidité revêt toute son importance lorsqu'on sait que cette hypoacidité est un signe de H gastrite atrophique qui prédispose au cancer; il est donc essentiel de savoir reconnaître les malades justiciables d'une surveillance. De plus, cette détermination permet de désigner les zones à biopsier, ou d'éviter une biopsie lorsque cette dernière n'est pas nécessaire.

Divers procédés ont été mis en oeuvre à ce jour pour déterminer le degré d'acidité de la muqueuse gastrique.

C'est ainsi que la pH-métrie a été employée; dans ce procédé une microélectrode de verre attachée à un fibroscope, ou bien passée dans ce fibroscope, permettait d'atteindre la muqueuse gastrique et d'en déterminer le pH. Un tel procédé est en voie d'abandon, car le matériel requis est très fragile, coûteux, d'un emploi mal aisé, et les mesures sont incertaines.

Dans un autre procédé, il a été envisagé d'utiliser un colorant, à savoir le rouge Congo pour déterminer le degré d'acidité de la muqueuse gastrique. Le protocole employé dans cette méthode est décrit dans un article de S. OKUDA, T. SAEGUSA, H. INUI et N. SENDA intitulé "an endoscopic method to investigate the gastric acid secretion" publié à la suite du Ier Congrès de la Société Internationale d'Endoscopie qui s'était déroulé à Tokyo en 1966.

Ce protocole est le suivant:

1 - Pré-traitement par administration d'atropine et de bromure de butylscopolamine.

2 - Injection sous cutanée de 30 mg d'antihistaminique (de type chlorhydrate de diphenhydramine), 15 à 30 minutes avant l'administration de stimulants décrits ci-après.

3 - Lavage de l'estomac avec une solution de bicarbonate de soude à 5 %.

4 - administration du colorant: 20 à 30 ml de rouge Congo à 0,3 % sont envoyés dans l'estomac à l'aide d'un cathéter, et sont appliqués sur la paroi gastrique en faisant changer le malade de position, ou bien en massant son abdomen.

5 - Injection sous cutanée de stimulants de la sécrétion de l'acidité gastrique.

6 - Observations endoscopiques à l'aide d'un fibroscope, ces observations devant se poursuivre entre 20 et 30 minutes après l'injection de stimulants, ce délai étant rendu nécessaire par le temps de réponse du colorant à la sécrétion de l'acidité gastrique: ledit colorant ne vire au bleu-noir que quand le pH s'abaisse de 5 à 2,8.

Un autre protocole, très proche du précédent, est décrit dans la revue Endoscopy 5 (1973) 61-69.

Dans l'un et l'autre cas, il est important de noter les contraintes auxquelles le malade est soumis avant et pendant l'endoscopie.

La présente invention concerne donc un agent de diagnostic pour la détermination du degré d'acidité de la muqueuse gastrique qui est un colorant, ledit procédé se distinguant de l'art antérieur en ce qu'il évite le lavage préliminaire de l'estomac et la stimulation de la sécrétion gastrique.

L'agent de diagnostic selon l'invention présente en outre l'avantage de donner une lecture immédiate, sans qu'il soit nécessaire de respecter le délai précédemment cité. Il évite donc de prolonger une épreuve pénible.

De façon plus spécifique, l'agent de diagnostic selon l'invention consiste à déterminer l'hypoacidité de la muqueuse gastrique en utilisant un colorant dont le changement de couleur permet une lecture immédiate en fibroscopie lorsqu'il entre en contact avec une zone hypoacide de ladite muqueuse gastrique.

Parmi les nombreux colorants existants, la demanderesse a sélectionné certains colorants, répondant aux différents critères qui étaient requis tant au niveau de la toxicité, puisque ce colorant est introduit directement dans l'estomac du malade, qu'à celui de son rôle d'indicateur coloré dont la couleur tranche sur la muqueuse quand elle est hypoacide.

Avantageusement, on utilisera parmi les colorants susmentionnés compatibles avec le procédé faisant l'objet de la présente invention le vert de bromocrésol de formule:

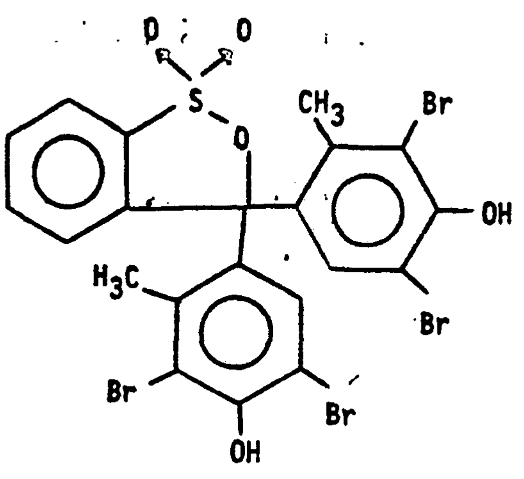

En effet, ce colorant présente tout d'abord l'avantage d'avoir une toxicité aigue nulle par voie orale, chez la souris mâle, en utilisant des doses de 300 mg/kg, en solution à 1 %.

En outre, ce colorant vire immédiatement au bleu-vert quand le pH s'élève de 3,5 à 5, cette couleur étant facilement repérable sur la muqueuse gastrique hypoacide.

Pratiquement, le vert de bromocrésol est utilisé en solution à 1/1000 dans l'éthanol et l'eau; il suffit alors de déposer quelques gouttes de cette solution sur la muqueuse à l'aide d'un cathéter passé dans le gastrofibroscope, pour noter une éventuelle hypoacidité de l'estomac, notamment dans le fundus. Eventuellement, cette solution contiendra de la glycérine ou toute autre substance qui favorise l'immobilisation sur place du réactif déposé.

Grâce à ce colorant, l'endoscopie n'est ni compliquée ni prolongée, et il n'est pas nécessaire de préparer l'estomac du malade ainsi que cela a été mentionné dans le cas du rouge Congo. Ce dernier produit est en outre moins favorable puisqu'en milieu hypoacide il est rouge, donc peu décelable sur la paroi de l'estomac, et que sa couleur bleu-noire, facilement décelable, n'intervient que pour un pH inférieur à 3, c'est-à-dire en milieu acide et non hypoacide.

D'autres indicateurs colorés peuvent également être utilisés à la place du vert de bromocrésol; c'est ainsi que le bleu de bromophénol (jaune à pH 3 et violet à pH 4,6) et le bleu de bromochlorophénol (jaune à pH 3 et bleu à pH 4,6) conviennent également, mais ils sont moins favorables, parce qu'ils virent à un pH plus bas que le vert de bromocrésol.

**Revendications**

1. Agent de diagnostic pour la détermination de l'hypoacidité gastrique au cours d'une fibroscopie, constitué d'une solution hydroalcoolique d'un indicateur coloré dont le virage se produit à un pH compris entre 3 et 5, tel que le vert de bromocrésol,le bleu de bromophénol ou le bleu de bromochlorophénol.
2. Agent de diagnostic selon la revendication 1, constitué d'une solution hydroalcoolique à 1/1000 de vert de bromocrésol.
3. Agent de diagnostic selon la revendication 2, caractérisé en ce que la solution comprend en outre de la glycérine.

**Patentansprüche**

1. Diagnostisches Mittel für die Bestimmung der gastrischen Hypoacidität im Verlauf einer Fibroskopie, bestehend aus einer wässrig-alkoholischen Lösung eines gefärbten Indikators, dessen Umschlagpunkt bei einem pH-Wert zwischen 3 und 5 liegt, wie Bromkresolgrün, Bromphenolblau oder Bromchlorphenolblau.
2. Diagnostisches Mittel nach Anspruch 1, bestehend aus einer wässrig-alkoholischen 1/1000-Lösung von Bromkresolgrün.
3. Diagnostisches Mittel nach Anspruch 2, dadurch gekennzeichnet, daß die Lösung zusätzlich Glycerin enthält.

**Claims**

1) Diagnostic agent for the detection of gastric hypoacidity during gastroscopy, which consists of a hydro-alcoholic solution of an indicator changing colour for a pH value between 3 and 5, such as bromcresol green, bromphenol blue or bromchlorphenol blue.

2) Diagnostic agent according to claim 1, which consists of bromcresol green in a 1/1000 hydro-alcoholic solution.

3) Diagnostic agent according to claim 2, which further comprises glycerin.